# EUROPÄISCHE PATENTSCHRIFT

(11) **EP 1 159 270 B1**
(45) Veröffentlichungstag und Bekanntmachung des Hinweises auf die Patenterteilung: **05.11.2003**
(21) Anmeldenummer: 00901555.3
(22) Anmeldetag: 13.01.2000
(51) Int. Cl.: C07D 213/50, C07D 213/57, C07D 213/52

(54) **VERFAHREN ZUR HERSTELLUNG VON 1-(6-METHYLPYRIDIN-3-YL)-2- 4-(METHYLSULFONYL PHENYL)ETHANON**
METHOD OF PREPARING 1-(6-METHYLPYRIDINE-3-YL)-2- 4-(METHYLSULFONYL)PHENYL ETHANONE
PROCEDE DE PRODUCTION DE 1-(6-METHYLPYIDIN-3-YL)-2- 4-(METHYLSULFONYL)PHENYL ETHANONE

(30) Priorität: 14.01.1999 EP 99100590; 29.07.1999 US 145996 P
(43) Veröffentlichungstag der Anmeldung: 05.12.2001
(73) Patentinhaber: Lonza AG, 4052 Basel (CH); Merck & Co., Inc., Rahway, New Jersey 07065-0907 (US)
(72) Erfinder: ARMBRUSTER, Erich, CH-3904 Naters (CH); BESSARD, Yves, CH-3960 Sierre (CH); KUO, David, Radnor, PA 19057 (US); LERESCHE, James, Edward, CH-3930 Visp (CH); PROPLESCH, Ralf, CH-3931 Eyholz (CH); RODUIT, Jean-Paul, CH-3979 Grône (CH)
(74) Vertreter: Ritthaler, Wolfgang, Dr.rer.nat.Dipl.-Chem.
(86) Internationale Anmeldenummer: EP0000240
(87) Internationale Veröffentlichungsnummer: WO00042014

(56) Entgegenhaltungen:
- WO-A-98/47871
- WO-A-99/15503
- WO-A-99/55830
- FR-A- 2 074 674
- US-A- 3 313 683
- US-A- 4 155 909
- US-A- 5 596 008
- BRAY B L ET AL: "SYNTHESIS OF ACYLPYRROLES VIA ALPHA-(DIMETHYLAMINO)-ALPHA-PYRROLYLACETON ITRILES" JOURNAL OF ORGANIC CHEMISTRY,US,AMERICAN CHEMICAL SOCIETY, Bd. 53, Nr. 26, 1988, Seiten 6115-6118, XP000886363
- BADIA D ET AL: "UNSYMMETRICALLY SUBSTITUTED DEOXYBENZOINS; AN IMPROVED PREPARATIVE ROUTE" BULLETIN DES SOCIETES CHIMIQUES BELGES,GB,PERGAMON PRESS, Bd. 98, Nr. 1, 1989, Seiten 77-81, XP000886342
- MARCH J.: "Organic Chemistry: Reactions, Mechanisms, and Structure" 1992 , WILEY , NEW YORK, US XP002135946 *4. Auflage, Kapitel 6-49, Seiten 964-965; Kapitel 6-50, Seite 965*
- KLINGSBERG E.: "Pyridine and its Derivatives" 1964 , WILEY , NEW YORK, US XP002135944 *Teil 4, Kapitel 14, Seite 137, Abschnitte b und c*
- MARCH J.: "Organic Chemistry: Reactions, Mechanisms, and Structure" 1992 , WILEY , NEW YORK, US XP002135945 *4. Auflage, Kapitel 6-12, Seite 895*

## Beschreibung

Die Erfindung betrifft ein Verfahren zur Herstellung von 1-(6-Methylpyridin-3-yl)-2-[(4-(methylsulfonyl)phenyl]ethanon der Formel 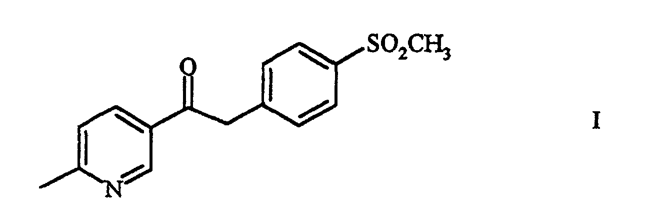 1-(6-Methylpyridin-3-yl)-2-[(4-(methylsulfonyl)phenyl]ethanon ist ein Ausgangsprodukt zur Herstellung von sogenannten COX-2-Inhibitoren, pharmazeutischen Wirkstoffe mit schmerzund entzündungshemmender Wirkung (R.S. Friesen et al., Bioorganic & Medicinal Chemistry Letters 8 (1998) 2777- 2782; WO 98/03484).

Die älteren internationalen Patentanmeldungen WO-A-99/15503 und WO-A-99/55830 beschreiben jeweils mehrstufige Verfahren zur Herstellung von 1-(6-Methylpyridin-3-yl)-2-[(4-(methylsulfonyl)phenyl]ethanon, wobei das gewünschte Endprodukt in der letzten Stufe durch Oxidation des entsprechenden Ketosulfids erhalten wird.

Die Aufgabe der vorliegenden Erfindung bestand darin, ein neues, technisch gangbares Verfahren zur Herstellung dieses Ausgangsprodukts bereitzustellen.

Diese Aufgabe wurde durch ein Verfahren zur Herstellung von 1-(6-Methylpyridin-3-yl)-2-[(4-(methylsulfonyl)phenyl]ethanon gelöst, worin 2-Methyl-5-vinylpyridin durch Umsetzung mit Ozon und anschliessende Reduktion zunächst in 2-Methylpyridin-5-carbaldehyd überführt wird, dieser dann mit einem Dialkylamin und einer Cyanverbindung zu einem N,N-Dialkylamino-(6-methyl-3-pyridyl)acetonitril der allgemeinen Formel 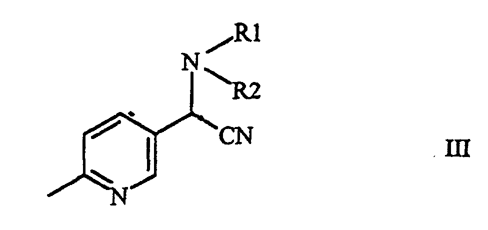 worin R¹ und R² gleich oder verschieden sind und C₁₋₄-Alkyl bedeuten, umgesetzt wird, und dieses schließlich in Gegenwart einer Base mit einem 4-(Methylsulfonyl)benzylhalogenid zu 1-(6-Methylpyridin-3-yl)-2-[(4-(methylsulfonyl)phenyl]ethanon der Formel I umgesetzt wird.

In einer besonders vorteilhaften Ausführungsform gemäß Anspruch 17 ist das erfindungsgemäße Verfahren ein vierstufiges Verfahren, wobei
in einer ersten Stufe a) 2-Methyl-5-ethylpyridin bei 500°C bis 700°C in Gegenwart eines Katalysators in das 2-Methyl-5-vinylpyridin überführt wird,
in einer zweiten Stufe b) das 2-Methyl-5-vinylpyridin mit Ozon und anschließend durch Reduktion in den 2-Methylpyridin-5-carbaldehyd überführt wird,
in einer dritten Stufe c) der 2-Methylpyridin-5-carbaldehyd mit einem Dialkylamin und einer Cyanverbindung in das entsprechende N,N-Dialkylamino-(6-methyl-3-pyridyl)acetonitril überführt wird, und schliesslich
in einer letzten Stufe d) das N,N-Dialkylamino-(6-methyl-3-pyridyl)acetonitril in Gegenwart einer Base mit einem 4-(Methylsulfonyl)benzylhalogenid zum 1-(6-Methylpyridin-3-yl)-2-[(4-(methylsulfonyl)phenyl]ethanon als Endprodukt umgesetzt wird.

Ein beträchtlicher Vorteil dieser Ausführungsform des erfindungsgemässen Verfahrens beruht auf der Tatsache, dass vom grosstechnisch verfügbaren 2-Methyl-5-ethylpyridin ausgegangen werden kann.

### Stufe a):

Die Dehydrierung von 2-Methyl-5-ethylpyridin zum 2-Methyl-5-vinylpyridin ist literaturbekannt (z.B. A. Nenz et al., Hydrocarbon Processing, 47(11), 1968, 139-144; US-A-2,769,773).

Die Umsetzung verläuft bei 500°C bis 700°C, vorzugsweise bei 600°C bis 700°C, in Gegenwart einer Vielzahl von verschiedenen Katalysatoren. In der Regel gelangen Katalysatoren auf Basis von Siliciumdioxid, Silicagel, Eisenoxid, Zinkoxid, Chromoxid, Kupferchromit, Magnesiumoxid, Kaliumoxid, Aluminiumoxid oder Borphosphat, einzeln oder als Mischung, gegebenenfalls aufgebracht auf einen Träger zum Einsatz. Gute Resultate lassen sich u.a mit einem Zinkoxidkatalysator aufgebracht auf Bimsstein als Träger erzielen. Es ist ausserdem von Vorteil für die Umsetzung, das 2-Methylpyridin mit Wasserdampf oder einem Inertgas, vorzugsweise aber mit Wasserdampf, zu verdünnen .

Das 2-Methyl-5-vinylpyridin kann auf einfache Weise, z.B. durch Abtrennen der Wasserphase und anschliessende Wasserdampfdestillation oder durch Vakuumdestillation so gereinigt werden, dass es für die darauffolgende Stufe b) bereitgestellt werden kann.

### Stufe b):

Die Umsetzung mit Ozon erfolgt zweckmässig in Gegenwart einer Mineralsäure bei einer Temperatur von -20 °C bis 0 °C, bevorzugt bei einer Temperatur von -15 °C bis -5 °C. Als Mineralsäure eignet sich Schwefelsäure oder Phosphorsäure, im besonderen Masse Schwefelsäure. Als Reaktionsmedium eignet sich Wasser und /oder ein polares Lösungsmittel. Als polares Lösungsmittel kann ein C₁₋₆-Alkohol wie Methanol, Ethanol, Propanol, Butanol, Pentanol oder Hexanol eingesetzt werden. Bewährt haben sich insbesondere Mischungen von einem C₁₋₆-Alkohol, wie Methanol oder Ethanol mit Wasser. Der intermediär gebildete Ozonkomplex wird zur Gewinnung des 2-Methyl-5-carbaldehyds reduktiv, bevorzugt mit einem Alkalihydrogensulfit aufgearbeitet.

Geeignete Alkalihydrogensulfite sind das Natrium- oder das Kaliumhydrogensulfit. Es ist allerdings auch möglich, andere bekannte Reduktionsmittel wie z.B. Dimethylsulfid, Thioharnstoff oder Trimethylphosphit oder Wasserstoff in Gegenwart eines geeigneten Katalysators zu wählen.

Im Falle der bevorzugten reduktiven Aufarbeitung mit Alkalihydrogensulfit arbeitet man im wesentlich gleichen Milieu wie für die Ozonisierung und üblicherweise bei einer Temperatur von -20 °C bis 20 °C, bevorzugt von -10 °C bis 0 °C.

Abhängig von den weiteren Aufarbeitungsschritten kann der 2-Methylpyridin-5-carbaldehyd oder ein Addukt von Alkalihydrogensulfit mit dem 2-Methylpyridin-5-carbaldehyd gebildet werden, nämlich ein 1-Hydroxy-(6-methylpyridin-3-yl)methansulfonsäuresalz.

Wünscht man den 2-Methyl-5-carbaldehyd zu isolieren, kann eine selektive Extraktion des Reaktionsgemisches bei einem pH von etwa 4 bis 5 mit einem geeigneten organischen Lösungsmittel, wie z. B. mit Essigsäureethylester erfolgen. Alternativ, aber bevorzugt, kann zunächst ein Addukt von Alkalihydrogensulfit mit dem 2-Methylpyridin-5-carbaldehyd gebildet werden, welches daraufhin bei einem pH-Wert von etwa 10 in den 2-Methyl-5-carbaldehyd gespalten wird.

Besonders bevorzugt wird für die Weiterumsetzung in Stufe c) jedoch gleich das Addukt von Alkalihydrogensulfit mit dem 2-Methylpyridin-5-carbaldehyd eingesetzt. Damit kann eine Isolation des relativ instabilen 2-Methylpyridin-5-carbaldehyds elegant umgangen werden.

Die Addukte von Alkalihydrogensulfit mit dem 2-Methylpyridin-5-carbaldehyd sind neu und nicht literaturbekannt und damit ebenso wie das Verfahren zu ihrer Herstellung Gegenstand der Erfindung. Die Addukte haben die allgemeine Formel 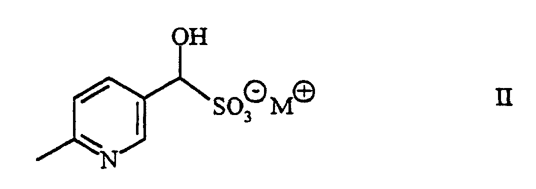 worin M ein Alkalimetall bedeutet, und werden als 1-Hydroxy-(6-methylpyridin-3-yl)methansulfonsäuresalze bezeichnet. Bevorzugte Alkalimetalle M sind Na und K.

### Stufe c):

Die Umsetzung des 2-Methylpyridin-5-carbaldehyds oder des Addukts von Alkalihydrogensulfit mit dem 2-Methylpyridin-5-carbaldehyd erfolgt nach dem Prinzip der Strecker-Synthese mit einer Cyanverbindung und einem Dialkylamin zum entsprechenden N,N-Dialkylamino-(6-methyl-3-pyridyl)acetonitril.

Als Cyanverbindung kann dabei eine wässerige HCN-Lösung oder eine wässerige Lösung von einem Alkalicyanid dienen. Besonders geeignete Dialkylamine sind C₁₋₄-Dialkylamin, worin C₁₋₄-Alkyl konkret Methyl, Ethyl, n-Propyl, iso-Propyl, n-Butyl, iso-Butyl oder tert.-Butyl bedeutet. Bevorzugte Dialkylamine sind Dimethylamin und Diethylamin.

Die Umsetzungstemperatur bewegt sich vorteilhaft im Bereich von 0 °C bis 30 °C.

Es kann von Vorteil sein, ein mit Wasser nicht mischbares Lösungsmittel, wie beispielsweise Toluol oder t-Butylmethylether, zuzusetzen. Die Aufarbeitung und Isolation des entsprechenden N,N-Dialkylamino-(6-methyl-3-pyridyl)acetonitrils kann dann über eine einfache Phasentrennung erfolgen. Die N,N-Dialkylamino-(6-methyl-3-pyridyl)acetonitrile der allgemeinen Formel 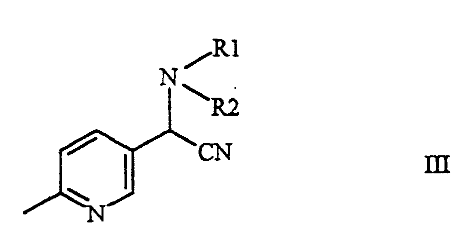 worin R¹ und R² gleich oder verschieden sind und C₁₋₄-Alkyl bedeuten, sind neue, nicht literaturbekannte Verbindungen und damit ebenso wie das Verfahren zu ihrer Herstellung Gegenstand der Erfindung.

C₁₋₄-Alkyl bedeutet wie oben Methyl, Ethyl, n-Propyl, iso-Propyl, n-Butyl, iso-Butyl oder tert.-Butyl. Bevorzugte Bedeutung von Alkyl ist Methyl oder Ethyl.

### Stufe d):

Die Überführung des N,N-Dialkylamino-(6-methyl-3-pyridyl)acetonitrils durch Reaktion mit dem 4-(Methylsulfonyl)benzylhalogenid zum Endprodukt der Formel I erfolgt in Gegenwart einer Base. Ein bevorzugtes 4-(Methylsulfonyl)benzylhalogenid ist das 4-(Methylsulfonyl)benzylchlorid.

Als Base kann dabei eine wässrige Alkalihydroxidlösung, bevorzugt eine wässrige Natriumhydroxid-Lösung eingesetzt werden, wobei in diesem Fall die Gegenwart eines üblichen Phasentransferkatalysators von Nutzen ist. Geeignete Phasentransferkatalysatoren sind z. B. die Tetraalkylammoniumhalogenide wie z. B. das Tetra-n-butylammoniumchlorid oder das Tetra-n-butylammoniumbromid. Die Umsetzungstemperatur bewegt sich dabei im Bereich von 40 °C bis 70 °C. Es kann von Vorteil sein, ein mit Wasser nicht mischbares Lösungsmittel, wie beispielsweise Toluol, Methylenchlorid oder t-Butylmethylether zuzusetzen.

Alternativ und bevorzugt kann als Base auch ein Alkalialkoholat verwendet werden. Geeignete Alkalialkoholate sind z. B. das Natrium- oder das Kalium-tert-butanolat oder das Natrium-tert-pentylat, bevorzugt aber das Kalium-tert-butanolat. Als Lösungsmittel empfehlen sich Ether wie z. B. das Tetrahydrofuran. Die Reaktionstemperatur bei dieser Variante beträgt in der Regel 15°C bis 25 °C.

Das 1-(6-Methylpyridin-3-yl)-2-[(4-(methylsulfonyl)phenyl]-ethanon kann auf fachmännisch übliche Weise, z. B. durch Ansäuern des Reaktionsgemisches und anschliessende Extraktion mit z. B. Toluol, isoliert werden. Eine weitere Aufreinigung kann durch Rekristallisation z. B. in Acetonitril, erfolgen.

### Beispiel 1

### Herstellung von 2-Methyl-5-vinylpyridin

Bimsstein mit einer Korngrösse von 6 bis 8 mm wird mit Wasser befeuchtet und mit 25% seines Trockengewichts mit Zinkoxid in Pulverform vermischt, in feuchtem Zustand in den Reaktor (Rohrlänge 750mm, Rohrdurchmesser 60mm) gefüllt und im Stickstoffstrom bei 650°C bis 700°C während 24h belassen.
76ml/h 2-Methyl-5-ethylpyridin wurden zusammen mit 87ml/h Wasserdampf bei 670°C bis 680°C und 665 mbar über den vorgenannten Katalysator geleitet. Am Reaktorende wurde ein Produktstrom bestehend aus 40,6 Gew.% 2-Methyl-5-vinylpyridin und 56,3% 2-Methyl-5-ethylpyridin entnommen. Bezogen auf umgesetztes 2-Methyl-5-ethylpyridin wurde eine Ausbeute von 93,0% erreicht.
Zur Reindarstellung des 2-Methyl-5-vinylpyridins wurde das Produktgemisch anschliessend wasserdampfdestilliert (266mbar, Kopftemperatur 59°C-60°C).
Die Reinigung von 2-Methyl-5-vinylpyridin kann auch mittels Vakuumdestillation (20 mbar, Temperatur ca. 90 °C) erfolgen.

### Beispiel 2

### Herstellung von 2-Methylpyridin-5-carbaldehyd

11,92 g 2-Methyl-5-vinylpyridin (Gehalt 85 %, 85 mmol), 50 ml Methanol und 10 ml Wasser wurden vorgelegt. Konzentrierte Schwefelsäure (9,81 g, 98 mmol) wurde so zudosiert, dass die Temperatur 20 °C nicht überstieg. Die Lösung wurde auf-12 °C gekühlt, anschliessend wurde ein Ozon/Sauerstoff Gemisch (ca. 5 % O₃. in O₂, 50 L/h) solange eingeleitet, bis das 2-Methyl-5-vinylpyridin vollständig umgesetzt wurde. Wasser (50 ml) und 40 % wässerige NaHSO₃ Lösung (22,7 g, 85 mmol) wurden vorsichtig zudosiert. Das Reaktionsgemisch wurde auf 20 °C erwärmt und mit 30 % NaOH (ca. 32 g, 0,24 mol) neutralisiert. Methanol wurde bei 30 - 40 °C abdestilliert, dann wurde zur Bildung des Bisulfit-Addukts nochmals 22,7 g 40 % NaHSO₃ Lösung zugegeben. Nach 30 min. Rühren wurde der pH wieder neutral gestellt, anschliessend wurden die neutralen Verunreinigungen mit 35 ml t-Butylmethylether extrahiert. Die wässerige Phase wurde mit 30 % NaOH auf pH 10 gebracht, und 26,5 g Na₂CO₃ (0,25 mol) wurden zugegeben. Der freigesetzte Aldehyd wurde mit 2 mal 80 ml t-Butylmethylether extrahiert. Nach Einengen des Lösungsmittels wurden 9 g 2-Methylpyridin-5-carbaldehyd als leicht gelbliches Öl erhalten.

| | |
|---|---|
| ¹H-NMR (CDCl₃) | 2,66 (s, 3H); |
| | 7,35 (d, *J* = 8 Hz, 1H); |
| | 8,07 (dd, *J* = 8 Hz and 2,1 Hz, 1 H); |
| | 8,96 (d, *J* = 2,1 Hz, 1 H); |
| | 10,08 (s, 1H). |
| ¹³C-NMR (CDCl₃) | 24,98 (CH3); |
| | 123,72 (C-5); |
| | 129,32 (C-3); |
| | 135,88 (C-4); |
| | 151,87 (C-2); |
| | 164,87 (C-6); |
| | 190,51 (C=O). |

### Beispiel 3a.

### Herstellung von N,N-Diethylamino-(6-methylpyridin-3-yl) acetonitril (ex 2-Methylpyridin-5-carbaldehyd)

73,2 g (1,25 eq.) Diethylamin und 100,3 g (1,15 eq.) einer 25 % HCN Lösung wurden bei 10 °C bis 15 °C simultan und während einer Stunde zu einer gut gerührten Mischung von 98,3 g (1,0 eq.) 2-Methylpyridin-5-carbaldehyd in 200 ml Wasser und 200 ml Toluol zugegeben. Das Reaktionsgemisch wurde während 3 h bei 30 °C gerührt.
Die Phasen wurden darauf getrennt und die Wasserphase mit 2 mal 100 ml Toluol extrahiert. Die organischen Phasen wurden zusammengenommen und dann das Toluol entfernt, worauf das Titelprodukt in Form eines gelblichen Öls und in einer Ausbeute von 172,3g (90,1 %) resultierte.

| | |
|---|---|
| ¹H-NMR (CDCl₃) | 8,65 (1H, s); |
| | 7,75 (1H, d); |
| | 7,20 (1H, d); |
| | 5,00 (1H, s); |
| | 2,68 (2H, m); |
| | 2,59 (13H, s); |
| | 2,50 (2H, m); |
| | 1,10 (6H, t). |
| ¹H-NMR (D₆-DMSO) | 8,50 (1H, s); |
| | 7,70 (1H, d); |
| | 7,32 (1H, d); |
| | 5,45 (1H, s); |
| | 2,58 (2H, m); |
| | 2,50 (3H, s); |
| | 2,40 (2H, m); |
| | 1,02 (6H, t). |

### Beispiel 3b.

### Herstellung von N,N-Diethylamino-(6-methylpyridin-3-yl) acetonitril (via Addukt von 2-Methylpyridin-5-carbaldehyd mit Natriumhydrogensulfit)

Die Ozonolyse wurde durchgeführt wie in Beispiel 2, ausgehend von 23,84 g 2-Methyl-5-vinylpyridine (83,1 % GC, 166,2 mmol). Nachdem die Verunreinigungen bei neutralem pH extrahiert worden waren, wurde die wässerige Phase auf 15 °C gekühlt und 21,94 g Diethylamin (0,3 mol), dann 9,8 g NaCN (0,2 mol) zugegeben (je 10 Min. Zugabezeit). Die Lösung wurde 4,5 h bei 15 °C gerührt und das Produkt wurde anschliessend mit 3 mal 85 ml Toluol extrahiert.
Die vereinigten Extrakte wurden eingeengt. Erhalten: 37,4 g N,N-Diethylamino-(6-methylpyridin-3-yl)acetonitril als oranges Öl. Gehalt : 83,7 % (GC, Gewicht-%), 0,34 % Aldehyd). Ausbeute : 92,7 % bezogen auf 2-Methyl-5-vinylpyridin.

| | |
|---|---|
| ¹H-NMR (CDCl₃) | 1,08 (t, 6H); |
| | 2,50 (m, 2H); |
| | 2,58 (s, 3H); |
| | 2,65 (m, 2H); |
| | 5,00 (s, 1H); |
| | 7,18 (d, *J* = 8 Hz, 1H); |
| | 7,74 (dd, *J* = 8 Hz, 2 Hz, 1H); |
| | 8,66 (d, *J* = 2 Hz, 1H). |

### Beispiel 3c.

### Herstellung und Charakterisierung des Addukts von 2-Methylpyridin-5-carbaldehyd mit Natriumhydrogensulfit

Nach der Bisulfit-Zugabe wurde eine Probe mit ¹H- und ¹³C-NMR gemessen. Die NMR-Signale des Aldehyds waren total verschwunden, stattdessen wurden die folgenden Signale beobachtet:

| | |
|---|---|
| ¹H-NMR (DMSO-d₆) | 1,96 (s, 3H); |
| | 5,01 (s, 1H); |
| | 6,85 (d, *J* = 8 Hz, 1H); |
| | 7,45 (dd, *J* = 8 and 2 Hz, 1H); |
| | 7,93 (d, *J* = 2 Hz, 1H). |
| ¹³C-NMR (DMSO-d₆) | 20,23 (CH3); |
| | 81,78 (CH); |
| | 124,14 (C-5); |
| | 130,02 (C-3); |
| | 138,76 (C-4); |
| | 143,08 (C-2); |
| | 156,04 (C-6). |

### Beispiel 4a.

### Herstellung von 1-(6-Methylpyridin-3-yl)-2-[(4-(methylsulfonyl)phenyl]-ethanon (wässrige NaOH als Base)

41,07g (89,1%, 1,00eq.) N,N-Diethylamino-(6-methylpyridin-3-yl) acetonitril, 30ml Toluol und 10,0g Cellite wurden vorgelegt. 72g (5eq.) einer 50% wässrigen NaOH-Lösung wurden darauf während 15 Minuten so zugegeben, dass die Temperatur auf 20°C gehalten werden konnte. Das Reaktionsgemisch wurde auf 45°C erwärmt. Unter starkem Rühren wurde eine erste Portion von 0,32g Tetra-n-butylammoniumbromid zugegeben.
Unmittelbar darauf gab man während 1,5h eine Lösung 0,32g Tetra-n-butylammoniumbromid und 44,52g (1,2eq.) 4-(Methylsulfonyl)benzylchlorid in 200ml Toluol zu. Nach der Hälfte der Zugabe wird eine dritte Portion von 0,32g Tetra n-butyl ammonium bromid zugegeben und während 6h bei 45°C weitergerührt.
Das Reaktionsgemisch wurde darauf auf Raumtemperatur gebracht, dann wurden 100ml Wasser und 100ml Toluol zugegeben. Nach Filtration und Waschen des Rückstands mit 25 ml Toluol wurden die Phasen getrennt. Die Wasserphase wurde mit 2 mal 50 ml Toluol extrahiert. Die kombinierten organischen Phasen wurden darauf mit 380 ml 1N HCl extrahiert. Neutralisation mit 29,6g 50% wässriger NaOH-Lösung bis pH 4,5 führte zur Auskristallisation des Titelprodukts. Die Suspension wurde filtriert, das Produkt mit 2 mal 100ml Wasser und 2 mal 80 ml Isopropanol/Wasser 1:1 gewaschen und anschliessend bei 20°C / 20mbar getrocknet.
Man erhielt 40,19 g (76,4%) des Titelproduktes mit einem Gehalt von 99,0%.
Fp. 182°C-183°C

| | |
|---|---|
| ¹H-NMR (CDCl₃) | 9,15 (1H, s); |
| | 8,18 (1H, d); |
| | 7,92 (2H, d); |
| | 7,47 (2H, d); |
| | 7,30 (1H, d); |
| | 4,39 (2H, s); |
| | 3,04 (3H, s); |
| | 2,63 (3H, s). |

### Beispiel 4b.

### Herstellung von 1-(6-Methylpyridin-3-yl)-2-[(4-(methylsulfonyl)phenyl]ethanon (Alkoholat, wasserfrei)

48,16g (84,5%, 1,00eq.) N,N-Diethylamino-(6-methylpyridin-3-yl) acetonitril in 20 ml Tetrahydrofuran wurden während 30 Minuten bei 20°C zu einer Suspension von 38,58g (1,7 eq.) Kalium-t-butanolat in 60 ml Tetrahydrofuran zugegeben. Unmittelbar anschliessend wurden 42,59g (1,03 eq) 4-(Methylsulfonyl)benzylchlorid in 60 ml Tetrahydrofuran während 1,5h bei 20°C bis 25°C zugegeben.
Nach 0,5h Rühren bei 20°C wurde das Reaktionsgemisch mit 100ml Wasser verdünnt und mit 180ml 2N HCl während einer Stunde auf pH 2 gebracht. Nach weiteren 0,5h bei 20°C wurde mit 10g einer 30% wässrigen NaOH-Lösung auf pH 3 gestellt. Nach einer Stunde Rühren bei 20 °C wurde die Suspension filtriert, das Produkt mit 2 mal 150 ml Wasser und 2 mal 100 ml Wasser/Isopropanol 1:1 gewaschen. Nach dem Trocknen bei 20°C/20mbar wurden 53,72g (92%) des Titelproduktes mit einem Gehalt von 99,1% erhalten.
Fp. 182°C-183°C

| | |
|---|---|
| ¹H-NMR (CDCl₃) | 9,15 (1H, s); |
| | 8,18 (1H, d); |
| | 7,92 (2H, d); |
| | 7,47 (2H, d); |
| | 7,30 (1H, d); |
| | 4,39 (2H, s); |
| | 3,04 (3H, s); |
| | 2,63 (3H, s). |

## Patentansprüche

1. Verfahren zur Herstellung von 1-(6-Methylpyridin-3-yl)-2-[(4-(methylsulfonyl)phenyl]-ethanon der Formel 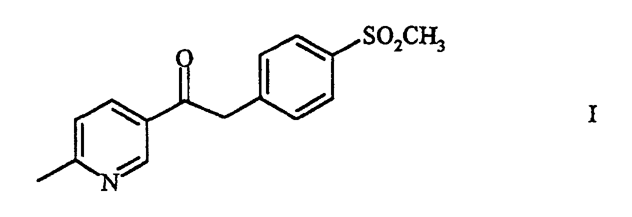 worin 2-Methyl-5-vinylpyridin durch Umsetzung mit Ozon und anschliessende Reduktion zunächst in 2-Methylpyridin-5-carbaldehyd überführt wird, dieser dann mit einem Dialkylamin und einer Cyanverbindung zu einem N,N-Dialkylamino-(6-methyl-3-pyridyl)acetonitril der allgemeinen Formel 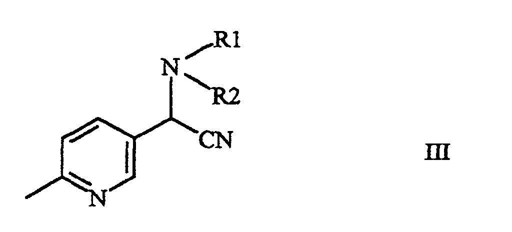 worin R¹ und R² gleich oder verschieden sind und C₁₋₄-Alkyl bedeuten, umgesetzt wird, und dieses schließlich in Gegenwart einer Base mit einem 4-(Methylsulfonyl)benzylhalogenid zu 1-(6-Methylpyridin-3-yl)-2-[(4-(methylsulfonyl)phenyl]ethanon der Formel I umgesetzt wird.

2. Verfahren gemäss Anspruch 1, worin die Umsetzung von 2-Methyl-5-vinylpyridin mit Ozon in Gegenwart einer Mineralsäure, bevorzugt bei einer Temperatur von -20 °C bis 0 °C, erfolgt.

3. Verfahren gemäss einem der Ansprüche 1 oder 2, worin die Reduktion mit einem Alkalihydrogensulfit unter Bildung eines 1-Hydroxy-(6-methylpyridin-3-yl)methansulfonsäuresalzes der allgemeinen Formel II 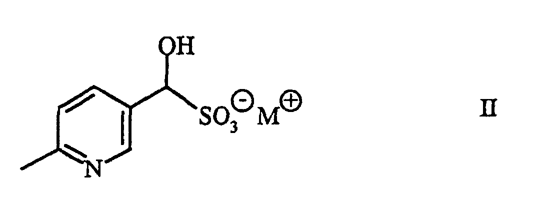 erfolgt, worin M ein Alkalimetall bedeutet.

4. Verfahren gemäss Anspruch 3, worin die Reduktion bei einer Temperatur von -20 °C bis 20 °C erfolgt.

5. Verfahren gemäss einem der Ansprüche 3 oder 4, worin das 1-Hydroxy-(6-methylpyridin-3-yl)methansulfonsäuresalz ohne es zu isolieren zur Herstellung des N,N-Dialkylamino-(6-methyl-3-pyridyl)acetonitrils der allgemeinen Formel III eingesetzt wird.

6. Verfahren gemäss einem der Ansprüche 1 bis 5, worin als Cyanverbindung bei der Umsetzung des 2-Methylpyridin-5-carbaldehyds eine wässrige HCN-Lösung oder eine wässrige Lösung eines Alkalicyanids verwendet wird.

7. Verfahren gemäss einem der Ansprüche 1 bis 6, worin die Temperatur für die Umsetzung des 2-Methylpyridin-5-carbaldehyds mit dem Dialkylamin und der Cyanverbindung 0 °C bis 30 °C beträgt.

8. Verfahren gemäss einem der Ansprüche 1 bis 7, worin als Base bei der Umsetzung des N,N-Dialkylamino-(6-methyl-3-pyridyl)acetonitrils der Formel III entweder eine wässrige Alkalihydroxidlösung zusammen mit einem Phasentransferkatalysator oder ein Alkalialkoholat in Gegenwart eines organischen Lösungsmittels verwendet wird.

9. Verfahren gemäß einem der Ansprüche 1 bis 8, worin das 2-Methyl-5-vinylpyridin ausgehend von 2-Methyl-5-ethylpyridin erhalten wird.

10. Verfahren gemäß Anspruch 9, worin 2-Methyl-5-ethylpyridin bei 500°C bis 700°C in Gegenwart eines Katalysators in das 2-Methyl-5-vinylpyridin überführt wird.

11. Verfahren gemäss Anspruch 10, worin als Katalysator ein Siliciumdioxid, Silicagel, Eisenoxid, Zinkoxid, Chromoxid, Kupferchromit, Magnesiumoxid, Kaliumoxid, Aluminiumoxid oder Borphosphat, einzeln oder als Mischung, gegebenenfalls aufgebracht auf einem Träger, verwendet wird.

12. Verfahren gemäss einem der Ansprüche 10 oder 11, worin die Umsetzung bei einer Temperatur von 600°C bis 700°C erfolgt.

13. 1-Hydroxy-(6-methylpyridin-3-yl)methansulfonsäuresalze der allgemeinen Formel 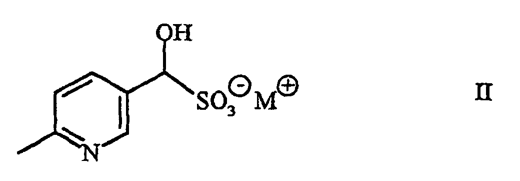 worin M ein Alkalimetall bedeutet.

14. Verfahren zur Herstellung von 1-Hydroxy-(6-methylpyridin-3-yl)methansulfonsäuresalzen gemäß Anspruch 13, worin 2-Methyl-5-vinylpyridin durch Umsetzung mit Ozon und anschliessende Reduktion mit einem Alkalihydrogensulfit in das Endprodukt der Formel II überführt wird.

15. N,N-Dialkylamino-(6-methyl-3-pyridyl)acetonitril der allgemeinen Formel 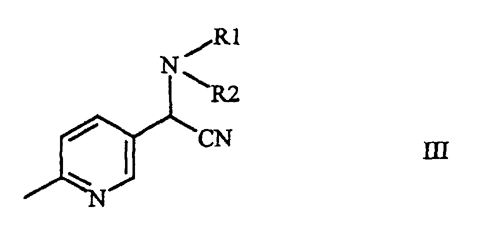 worin R¹ und R² gleich oder verschieden sind und C₁₋₄-Alkyl bedeuten.

16. Verfahren zur Herstellung eines N,N-Dialkylamino-(6-methyl-3-pyridyl)acetonitrils der allgemeinen Formel III gemäß Anspruch 15, worin 2-Methylpyridin-5-carbaldehyd oder 1-Hydroxy-(6-methyl-pyridin-3-yl)methansulfonsäuresalz der allgemeinen Formel II 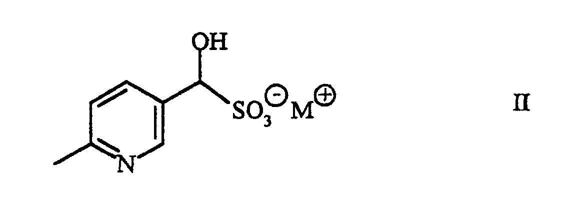 mit einem Dialkylamin und einer Cyanverbindung zum Endprodukt der Formel III umgesetzt werden.

17. Verfahren zur Herstellung von 1-(6-Methylpyridin-3-yl)-2-[(4-(methylsulfonyl)phenyl]-ethanon der Formel 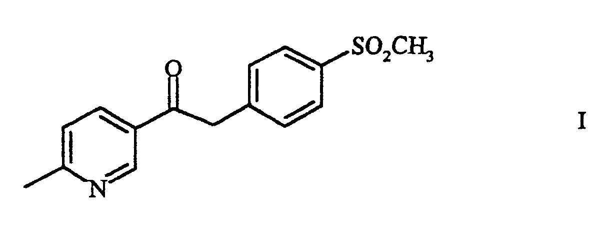 **dadurch gekennzeichnet, dass**
in einer ersten Stufe a) 2-Methyl-5-ethylpyridin bei 500°C bis 700°C in Gegenwart eines Katalysators in das 2-Methyl-5-vinylpyridin überführt wird,
in einer zweiten Stufe b) das 2-Methyl-5-vinylpyridin durch Umsetzung mit Ozon und anschliessende Reduktion in den 2-Methylpyridin-5-carbaldehyd überführt wird,
in einer dritten Stufe c) der 2-Methylpyridin-5-carbaldehyd mit einem Dialkylamin und einer Cyanverbindung in das entsprechende N,N-Dialkylamino-(6-methyl-3-pyridyl)acetonitril überführt wird, und schliesslich
in einer letzten Stufe d) das N,N-Dialkylamino-(6-methyl-3-pyridyl)acetonitril in Gegenwart einer Base mit einem 4-(Methylsulfonyl)benzylhalogenid zum 1-(6-Methylpyridin-3-yl)-2-[(4-(methylsulfonyl)phenyl]ethanon umgesetzt wird.

## Claims

1. Process for preparing 1-(6-methylpyridin-3-yl)-2-[4-(methylsulfonyl)phenyl]ethanone of the formula 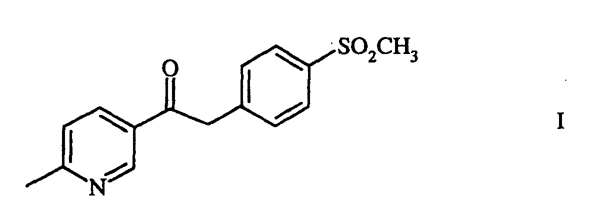 wherein 2-methyl-5-vinylpyridine is first converted, by reaction with ozone and subsequent reduction, to 2-methylpyridine-5-carbaldehyde, which is then reacted with a dialkylamine and a cyano compound to give a N,N-dialkylamino-(6-methyl-3-pyridyl)acetonitrile of the general formula 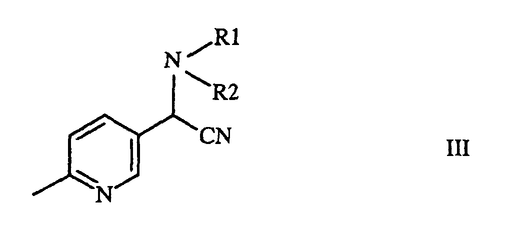 wherein R¹ and R² are the same or different and represent C₁₋₄-alkyl, which finally is reacted, in the presence of a base, with a 4-(methylsulfonyl)benzyl halide to give 1-(6-methylpyridin-3-yl)-2-[4-(methylsulfonyl)phenyl]-ethanone of formula I.

2. Process according to claim 1, wherein the reaction of 2-methyl-5-vinylpyridine with ozone is carried out in the presence of a mineral acid, preferably at a temperature of from -20°C to 0°C.

3. Process according to claim 1 or 2, wherein the reduction is carried out using an alkali hydrogen sulfite, with formation of a 1-hydroxy-(6-methylpyridin-3-yl)methane-sulfonic acid salt of the general formula II 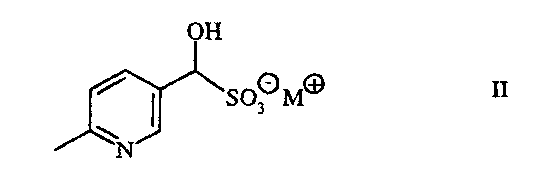 wherein M is an alkali metal.

4. Process according to claim 3, wherein the reduction is carried out at a temperature of from -20°C to 20°C.

5. Process according to claim 3 or 4, wherein the 1-hydroxy- (6-methylpyridin-3-yl)methanesulfonic acid salt is used without isolation for preparing the N,N-dialkylamino-(6-methyl-3pyridyl)acetonitrile of the general formula III.

6. Process according to any one of claims 1 to 5, wherein the cyano compound used in the reaction of the 2-methylpyridine-5-carbaldehyde is an aqueous HCN solution or an aqueous solution of alkali cyanide.

7. Process according to any one of claims 1 to 6, wherein the temperature for the reaction of the 2-methylpyridine-5-carbaldehyde with the dialkylamine and the cyano compound is from 0°C to 30°C.

8. Process according to any one of claims 1 to 7, wherein the base used in the reaction of the N,N-dialkylamino-(6-methyl-3-pyridyl)acetonitrile of the formula III is either an aqueous alkali hydroxide solution together with a phase-transfer catalyst or an alkali alkoxide in the presence of an organic solvent.

9. Process according to any one of claims 1 to 8, wherein the 2-methyl-5-vinylpyridine is obtained starting from 2-methyl-5-ethylpyridine.

10. Process according to claim 9, wherein 2-methyl-5-ethylpyridine is converted at from 500°C to 700°C in the presence of a catalyst to 2-methyl-5-vinylpyridine.

11. Process according to claim 10, wherein the catalyst used is silica, silica gel, iron oxide, zinc oxide, chromium oxide, copper chromite, magnesium oxide, potassium oxide, aluminium oxide or borophosphate, on its own or as a mixture, optionally applied to a support.

12. Process according to claim 10 or 11, wherein the reaction is carried out at a temperature of from 600°C to 700°C.

13. A 1-hydroxy- (6-methylpyridin-3-yl)methanesulfonic acid salt of the formula 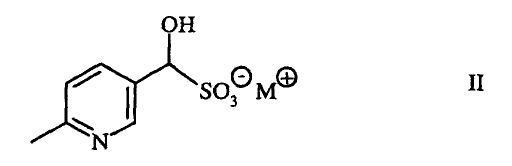 wherein M is an alkali metal.

14. Process for preparing 1-hydroxy-(6-methylpyridin-3-yl)methanesulfonic acid salts according to claim 13, wherein 2-methyl-5-vinylpyridine is converted, by reaction with ozone and subsequent reduction with an alkali hydrogen sulfite, to the end product of formula II.

15. A N,N-dialkylamino-(6-methyl-3-pyridyl)acetonitrile of formula 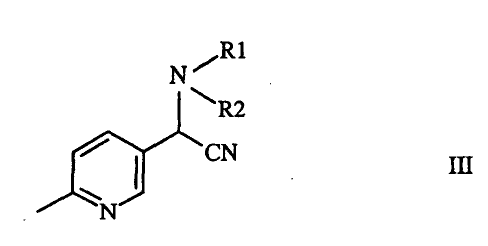 wherein R¹ and R² are the same or different and represent C₁₋₄-alkyl.

16. Process for preparing a N,N-dialkylamino-(6-methyl-3-pyridyl)acetonitrile of the general formula III according to claim 15, wherein 2-methylpyridine-5-carbaldehyde or a 1-hydroxy-(6-methylpyridin-3-yl)methanesulfonic acid salt of formula II 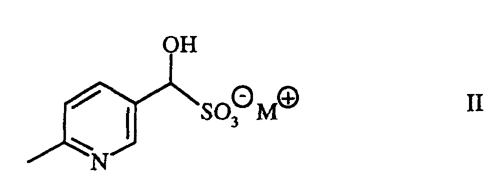 is reacted with a dialkylamine and a cyano compound to give the end product of the formula III.

17. Process for preparing 1-(6-methylpyridin-3-yl)-2-[4-(methylsulfonyl)phenyl]ethanone of formula 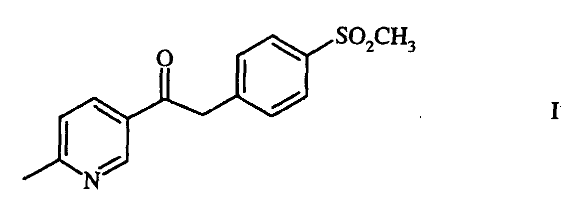 **characterized in that**
in a first step a), 2-methyl-5-ethylpyridine is converted at from 500°C to 700°C in the presence of a catalyst into 2-methyl-5-vinylpyridine,
in a second step b), the 2-methyl-5-vinylpyridine is converted, by reaction with ozone and subsequent reduction, to 2-methylpyridine-5-carbaldehyde,
in a third step c), 2-methylpyridine-5-carbaldehyde is converted, using a dialkylamine and a cyano compound, to the corresponding N,N-dialkylamino-(6-methyl-3-pyridyl)acetonitrile, and finally,
in a last step d), the N,N-dialkylamino-(6-methyl-3-pyridyl)acetonitrile is reacted in the presence of a base with a 4-(methylsulfonyl)benzyl halide to give 1-(6-methylpyridin-3-yl)-2-[4-(methylsulfonyl)phenyl]-ethanone.

## Revendications

1. Procédé de préparation de 1-(6-méthylpyridin-3-yl)-2-[(4-méthylsulfonyl)-phényl]éthanone de la formule 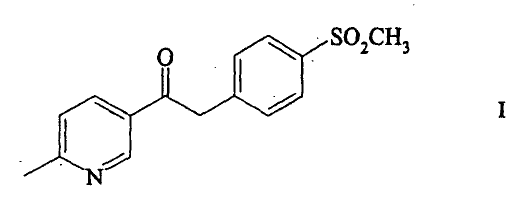 dans lequel de la 2-méthyl-5-vinylpyridine est tout d'abord convertie en 2-méthylpyridine-5-carbaldéhyde par réaction avec de l'ozone suivie d'une réduction, et ensuite ce dernier est mis à réagir avec une dialkylamine et un composé cyano pour donner un N,N-dialkylamino-(6-méthyl-3-pyridyl)-acétonitrile de la formule générale 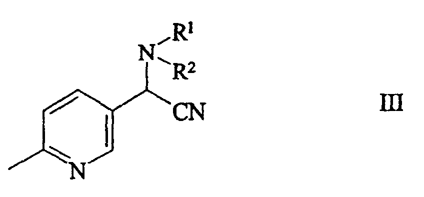 dans laquelle R¹ et R² sont identiques ou différents et représentent un alkyle en C₁ à C₄, et pour finir celui-ci est mis à réagir en présence d'une base mit un halogénure de 4-(méthylsulfonyl)benzyle pour donner la 1-(6-méthylpyridin-3-yl)-2-[(4-méthylsulfonyl)phényl]éthanone de la formule I.

2. Procédé selon la revendication 1, dans lequel la réaction de la 2-méthyl-5-vinylpyridine avec de l'ozone se fait en présence d'un acide minéral, de préférence à une température de -20°C à 0°C,.

3. Procédé selon l'une des revendications 1 ou 2, dans lequel la réduction se fait avec un hydrogénosulfite alcalin avec formation d'un sel de l'acide 1-hydroxy-(6-méthylpyridin-3-yl)méthanesulfonique de la formule générale II 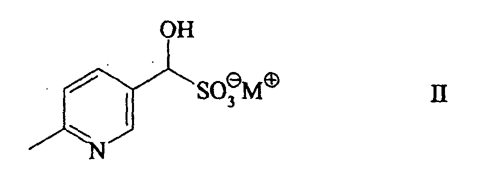 dans laquelle M représente un métal alcalin.

4. Procédé selon la revendication 3, dans lequel la réduction se fait à une température de -20°C à 20°C.

5. Procédé selon l'une des revendications 3 ou 4, dans lequel le sel de l'acide 1-hydroxy-(6-méthylpyridin-3-yl)méthanesulfonique est utilisé sans l'isoler dans la préparation du N,N-dialkylamino-(6-méthyl-3-pyridyl)acétonitrile de la formule générale III.

6. Procédé selon l'une des revendications 1 à 5, dans lequel le composé cyano utilisé dans la réaction du 2-méthylpyridine-5-carbaldéhyde est une solution aqueuse de HCN ou une solution aqueuse d'un cyanure alcalin.

7. Procédé selon l'une des revendications 1 à 6, dans lequel la température de la réaction du 2-méthylpyridine-5-carbaldéhyde avec la dialkylamine et avec le composé cyano se situe dans la plage de 0°C à 30°C.

8. Procédé selon l'une des revendications 1 à 7, dans lequel la base utilisée dans la réaction du N,N-dialkylamino-(6-méthyl-3-pyridyl)acétonitrile de la formule III est soit une solution aqueuse d'un hydroxyde alcalin ensemble avec un catalyseur de transfert de phase, soit un alcoolate alcalin en présence d'un solvant organique.

9. Procédé selon l'une des revendications 1 à 8, dans lequel la 2-méthyl-5-vinylpyridine est obtenue à partir de 2-méthyl-5-éthylpyridine.

10. Procédé selon la revendication 9, dans lequel la 2-méthyl-5-éthylpyridine est convertie en 2-méthyl-5-vinylpyridine à une température de 500°C à 700°C en présence d'un catalyseur.

11. Procédé selon la revendication 10, dans lequel le catalyseur utilisé est un dioxyde de silicium, un gel de silice, un oxyde de fer, un oxyde de zinc, un oxyde de chrome, un chromite de cuivre, un oxyde de magnésium, un oxyde de potassium, un oxyde d'aluminium ou un phosphate de bore, soit seul, soit sous la forme d'un mélange, le cas échéant déposé sur un support.

12. Procédé selon l'une des revendications 10 ou 11, dans lequel la réaction se fait à une température dans la plage de 600°C à 700°C.

13. Sels de l'acide 1-hydroxy-(6-méthylpyridin-3-yl)méthanesulfonique de la formule générale 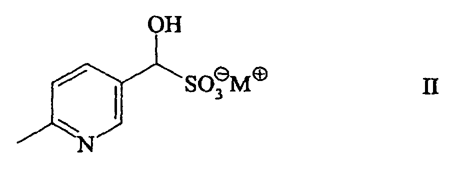 dans laquelle M représente un métal alcalin.

14. Procédé de préparation de sels de l'acide 1-hydroxy-(6-méthylpyridin-3-yl)-méthanesulfonique selon la revendication 13, dans lequel la 2-méthyl-5-vinylpyridine est convertie en produit final de la formule II par réaction avec de l'ozone suivie d'une réduction avec un hydrogénosulfite alcalin.

15. N,N-dialkylamino-(6-méthyl-3-pyridyl)acétonitrile de la formule générale 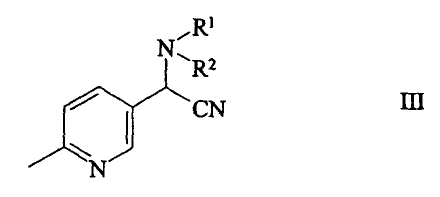 dans laquelle R¹ et R² sont identiques ou différents et représentent un alkyle en C₁ à C₄.

16. Procédé de préparation d'un N,N-dialkylamino-(6-méthyl-3-pyridyl)acétonitrile de la formule générale III selon la revendication 15, dans lequel du 2-méthylpyridine-5-carbaldéhyde ou un sel de l'acide 1-hydroxy-(6-méthylpyridin-3-yl)- méthanesulfonique de la formule générale II 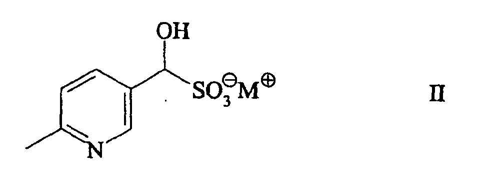 est mis à réagir avec une dialkylamine et un composé cyano pour donner le produit final de la formule III.

17. Procédé de préparation de 1-(6-méthylpyridin-3-yl)-2-[(4-méthylsulfonyl)-phényl]éthanone de la formule 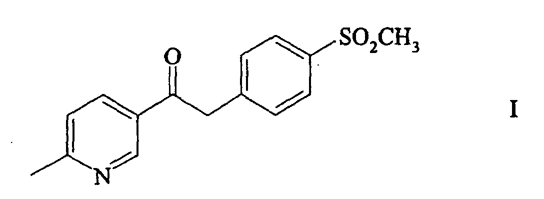 **caractérisé en ce que**
dans une première étape a), de la 2-méthyl-5-éthylpyridine est convertie en 2-méthyl-5-vinylpyridine à une température de 500°C à 700°C en présence d'un catalyseur,
dans une deuxième étape b), la 2-méthyl-5-vinylpyridine est convertie en 2-méthylpyridine-5-carbaldéhyde par réaction avec de l'ozone suivie d'une réduction,
dans une troisième étape c), le 2-méthylpyridine-5-carbaldéhyde est converti en N,N-dialkylamino-(6-méthyl-3-pyridyl)acétonitrile correspondant par réaction avec une dialkylamine et un composé cyano, et enfin
dans une dernière étape d), le N,N-dialkylamino-(6-méthyl-3-pyridyl)-acétonitrile est mis à réagir en présence d'une base avec un halogénure de 4-(méthylsulfonyl)benzyle pour donner la 1-(6-méthylpyridin-3-yl)-2-[(4-méthylsulfonyl)phényl]éthanone.
